# EUROPÄISCHE PATENTSCHRIFT

(11) **EP 2 812 154 B1**
(45) Veröffentlichungstag und Bekanntmachung des Hinweises auf die Patenterteilung: **14.10.2015**
(21) Anmeldenummer: 13700459.4
(22) Anmeldetag: 14.01.2013
(51) Int. Cl.: B25B 13/46, A61C 8/00, A61B 17/88

(54) **RATSCHE UND DEREN HERSTELLUNGSVERFAHREN SOWIE DREHMOMENTÜBERTRAGUNGSSYSTEM UND VERFAHREN ZUM ÜBERTRAGEN EINES DREHMOMENTS AUF EIN EINDREHWERKZEUG, UND VERWENDUNG EINER DERARTIGEN RATSCHE IM MEDIZINBEREICH**
RATCHET AND METHOD OF PRODUCING SAME, AS WELL AS A TORQUE TRANSMISSION SYSTEM AND METHOD FOR TRANSMITTING TORQUE TO AN IMPLANT DRIVER, AND USE OF SUCH A RATCHET IN THE MEDICAL FIELD
DISPOSITIF A CLIQUET ET SON PROCEDE DE FABRICATION AINSI QUE SYSTEME DE TRANSMISSION DE COUPLE ET PROCEDE PERMETTANT DE TRANSMETTRE UN COUPLE A UN OUTIL ROTATIF DE SERRAGE, ET UTILISATION D'UN TEL DISPOSITIF A CLIQUET DANS LE DOMAINE MEDICAL

(30) Priorität: 09.02.2012 DE 102012101050
(43) Veröffentlichungstag der Anmeldung: 17.12.2014
(73) Patentinhaber: Hipp Medical AG, 78600 Kolbingen (DE)
(72) Erfinder: WAIZENEGGER, Markus, 78600 Kolbingen (DE)
(74) Vertreter: Kuhnen & Wacker
(86) Internationale Anmeldenummer: PCT/EP2013/000093
(87) Internationale Veröffentlichungsnummer: WO 2013/117291

(56) Entgegenhaltungen:
- EP-A2- 1 138 441
- DE-A1- 1 782 406
- DE-U1-202007 015 689
- FR-A- 434 056
- FR-A- 488 360
- FR-A- 545 717
- FR-A1- 2 687 599
- FR-A1- 2 697 770
- US-A- 747 679

## Beschreibung

Die Erfindung betrifft eine Ratsche für den Medizinbereich nach dem Oberbegriff des Anspruchs 1, insbesondere eine Ratsche für chirurgische Eingriffe oder eine Dentalratsche. Die Erfindung betrifft ferner nach Anspruch 9 ein Drehmomentübertragungssystem sowie nach Anspruch 14 die Verwendung einer derartigen Ratsche im Medizinbereich. Die Erfindung betrifft darüber hinaus nach Anspruch 15 ein Verfahren zum Übertragen eines aufzubringenden Drehmoments mit einer derartigen Ratsche auf ein Eindrehwerkzeug und nach Anspruch 19 ein Verfahren zum Herstellen einer derartigen Ratsche.

Bei chirurgischen Eingriffen unter Verwendung von Werkzeugen und/oder medizinischen Geräten ist das Problem einer ausreichenden Sterilisation bekannt. Um zu vermeiden, dass Blut- oder Geweberückstände sowie sonstige Wundausscheidungen in Schlitze oder Verbindungsstellen von korrelierenden Bauteilen gelangen und sich dort ablagern können, wird im medizinischen Bereich Wert darauf gelegt, dass die zur Anwendung kommenden Werkzeuge aus möglichst wenig Einzelteilen bestehen.

Durch die Ausbildung der Werkzeuge aus möglichst wenig Teilen, insbesondere einteilig, ist es möglich, Infektionen, die durch Ablagerungen oder Keime hervorgerufen werden können, die sich an den Verbindungsstellen von mehrteilig ausgestalteten Werkzeugen befinden, weitestgehend zu reduzieren.

Ferner bedarf es für eine notwendige Sterilisation einteiliger, medizinischer Werkzeuge keiner Demontage einzelner Bestandteile, bei denen es zu möglichen Abnutzungen und Verschleißerscheinungen an den direkten Kontaktstellen kommen kann.

Werkzeuge, die aus wenigen Einzelteilen bestehen oder gar einteilig ausgebildet sind, zeigen ferner den Vorteil, dass eine fehlerhafte Zusammensetzung durch den Benutzer reduziert oder vermieden werden kann, insbesondere auch dann, wenn mehrere baugleiche Werkzeuge in einem Behältnis gereinigt werden.

Zudem kann der Arbeitsaufwand, welcher entsteht, wenn die einzelnen Bauteile mehrteiliger Werkzeuge zusammengefügt werden und im Anschluss daran die Funktionsfähigkeit des zusammengebauten Werkzeugs überprüft wird, verringert werden.

Während in vielen medizinischen Bereichen daher vorzugsweise Werkzeuge zum Einsatz kommen, die aus sehr wenigen, oftmals nur einem einzigen Teil bestehen, beispielsweise einstückig ausgebildete Zangen, Sägen, Bohrer und dergleichen, ist es im Bereich der Mund-, Kiefer- und Gesichtschirurgie nach wie vor der Fall, dass einige Spezialwerkzeuge mehrteilig ausgebildet sein müssen, um die gewünschte Funktion erfüllen zu können.

Dies ist vor allem im Bereich der Dental- und Implantationsbehandlung von besonderer Bedeutung, da beim Einsetzen von Implantatträgem oder Zahnsockeln in den Kiefer besonders darauf geachtet werden muss, keine Infektionserreger zu übertragen, die dann nicht nur das Zahnfleisch sondern unter Umständen sogar den Kieferknochen angreifen könnten. Derartige Implantatträger oder Zahnsockel werden dabei üblicherweise mittels mehrteilig ausgebildeter Dentalratschen in den Kieferknochen eingeschraubt.

Zur Vermeidung der vorgenannten Nachteile, insbesondere des mit der mehrteiligen Ausbildung von medizinischen Werkzeugen verbundenen Infektionsrisikos, sollen jedoch auch im Bereich der Mund-, Kiefer- und Gesichtschirurgie möglichst aus einem Teil gefertigte Werkzeuge, d.h. einteilig ausgebildete Dentalratschen, zum Einsatz kommen.

Aus der WO 2006/029542 A1 ist ein Drehmomentschlüssel für den Medizinbereich bekannt, welcher als Ratscheninstrument konzipiert ist. An der Peripherie der Aufnahmeöffnung ist ein begrenzt bewegliches Klinkensegment angeordnet, dessen Frontpartie zur Aufnahmeöffnung weist. Über einen auslenkbaren, linear-elastischen Biegeast wird bei Betätigung des Drehmomentschlüssels in Vorwärtsrichtung das zu generierende Drehmoment mittels einer vom Benutzer ausgeübten Vorwärtskraft eingebracht.

Vom Klinkensegment erstreckt sich eine Klinkenfeder in einen Halsbereich hinein. Das Klinkensegment und die Klinkenfeder bilden dabei eine aus mehreren Teilen zusammengesetzte Klinke, die alternativ auch einteilig ausgebildet werden kann. Das Klinkensegment und die Klinkenfeder sind in einem kanalförmigen Spielraum angeordnet, welcher die Auslenkung beider in der Ebene gegen die Kraft der Klinkenfeder erlaubt.

Im Bereich der Mund-, Kiefer- und Gesichtschirurgie ist es oftmals wichtig, das zu übertragende Drehmoment feinfühlig von der Dentalratsche zum Eindrehinstrument zu übertragen. Dies ermöglicht jedoch der Drehmomentschlüssel der WO 2006/029542 A1 nicht, da die Drehmomentenübertragung erst dann Eintritt, wenn das Klinkensegment mit dem oberen ersten Anschlag in Kontakt kommt. Somit ist eine sensible Übertragung des Drehmoments auf das Eindrehinstrument nicht möglich.

In der DE 20 2007 015 689 U1 wird eine Dentalratsche vorgeschlagen, bei der ein so bezeichneter Mono-Ratschenkopf am Griffstück angeordnet ist. Der Mono-Ratschenkopf sieht dabei die Umfassung für das Eindrehwerkzeug als Federelement mit Klinkenelement vor. Die biegeelastische Umfassung ist ferner ringförmig mit einer Unterbrechung bzw. einem Spalt ausgebildet, so dass sie eine Form eines offenen Rings aufweist. An einem freien Ende der Umfassung bzw. des offen Rings ist ein Klinkenelement ausgebildet. Das Klinkenelement lässt das Bewegen des Eindrehwerkzeugs in nur eine Richtung zu. Die andere Drehrichtung wird durch Einrasten des Eindrehwerkzeugs in das Klinkenelement erreicht.

Ein weiterer wesentlicher Bestandteil der Dentalratsche der DE 20 2007 015 689 U1 ist die nutenförmige Konturausbildung des Eindrehwerkzeugs, wie sie z.B. auch bei einem Torx vorkommt. Nur mit dieser Ausgestaltung ist es mit der Ratsche der DE 20 2007 015 689 U1 möglich, die gewünschte Funktionsweise zu erreichen.

Eine dosierende Übertragung des Drehmoments auf das Eindrehwerkzeug ist mit der Dentalratsche der DE 20 2007 015 689 U1 ebenfalls nicht möglich.

Dementsprechend ist es Aufgabe der vorliegenden Erfindung, eine Ratsche zu schaffen, mit der das aufzubringende Drehmoment fein dosiert auf ein Eindrehwerkzeug aufbringbar ist.

Diese Aufgabe wird durch eine Ratsche mit den Merkmalen des Anspruchs 1 gelöst.

Erfindungsgemäß wird dabei eine Ratsche vorgeschlagen, die ein Griffstück und ein daran angeordnetes Drehpunktgehäuse, das als eine ebene Spirale ausgebildet ist, aufweist. Ferner ist die Spirale biegeelastisch ausgebildet und die Ratsche ist derart betätigbar, dass sich die Spirale in einer im Wesentlichen senkrecht zur Drehachse orientierten Ebene eindreht, wobei die Spirale als eine logarithmische Spirale, eine hyperbolische Spirale oder eine archimedische Spirale ausgebildet ist.

Durch die Ausgestaltung des Drehpunktgehäuses als biegeelastische Spirale ist es auf einfache Weise möglich, dass bei der Drehung der Ratsche in Drehmomentübertragungsrichtung ein Teil der von einem Benutzer aufzubringenden Kraft in die biegeelastische Verformung der Spirale fließt und der andere Teil das auf ein Eindrehwerkzeug zu übertragende Drehmoment verursacht. Zunächst verursacht die vom Benutzer auf das Griffstück aufgebrachte Kraft eine biegeelastische Verformung der Spirale bis das Eindrehwerkzeug von der Spirale aufgrund der zwischen den Elementen auftretenden Reibung kraftschlüssig umschlossen ist, sodass anschließend die weitere vom Benutzer aufgebrachte Kraft die Drehmomentübertragung auf das Eindrehwerkzeug verursacht. Wird die zusätzlich vom Benutzer aufgebrachte Kraft hingegen zu groß, was z.B. zu einer Beschädigung von Kiefer, Implantatträger, Zahnsockel oder dergleichen führen könnte, schlupft bzw. dreht sich die Ratsche bezüglich dem Eindrehwerkzeug durch. Somit kann die Drehmomentübertragung dosierend auf das Eindrehwerkzeug übertragen werden, ohne den menschlichen Körper oder andere Komponenten zu beschädigen.

Die Verformung der biegelastischen Spirale erfolgt durch Eindrehen selbiger in einer Ebene, d.h. bei Betrachtung der Draufsicht (zweidimensional) dreht sich die Spirale bei einer Drehmomentübertragung um eine Drehachse des Drehpunktgehäuses, also nach Innen ein. Die Biegeelastizität der Spirale erstreckt sich somit lediglich über eine Ebene. Bei Drehung der Ratsche in Freilaufrichtung dreht sich die Spirale von der Drehachse weg, mit anderen Worten sie öffnet sich.

Darüber hinaus drückt eine Federkraft, welche durch die Ausgestaltung des Drehpunktgehäuses als Spirale bedingt ist, mit einer vordefinierten Anpresskraft die Spirale bei Drehung in Drehmomentübertragungsrichtung zusätzlich auf das Eindrehwerkzeug. Die Kombination von Oberflächenrauheit der Spirale und Anpressdruck der Spirale an das Eindrehwerkzeug erhöht demzufolge die Drehmomentenübertragung wirksam.

Ferner kann durch einfaches Wenden der Ratsche bezüglich einer Längsachse des Griffstücks die Drehmomentübertragungsrichtung verändert werden, nämlich in eine Freilaufrichtung vor dem Wendevorgang. So kann auf vorteilhafte Weise die Drehmomentübertragungsrichtung der Ratsche bestimmt werden, ohne die Ratsche aufwendig umzurüsten.

Mit der erfindungsgemäß konstruktiv einfachen Ausgestaltung der Ratsche können die Herstellkosten gering gehalten werden.

Schließlich ist die Ratsche auch einfach und ohne großen Aufwand sogar von weniger erfahrenem Personal, beispielsweise Auszubildenden beim Zahnarzt, die mit der Desinfektion der verwendeten Werkzeuge und der Vorbereitung des Behandlungsraumes vertraut gemacht werden, handhabbar.

Ist die Spirale als archimedische Spirale ausgebildet, kann die Spirale das Eindrehwerkzeug fest umschließen, da der Radius der Spirale ab der Verbindung zwischen Spirale und Griffstück bis zu dem freien Ende der Spirale proportional zum Winkel abnimmt. Somit wird die Federkraft der Spirale auf das Eindrehwerkzeug wirksam erhöht. Je nach Bedarf der Federkraft kann die Spirale auch als logarithmische oder hyperbolische Spirale ausgebildet sein.

Weitere Ausgestaltungen der erfindungsgemäßen Ratsche sind Gegenstand der abhängigen Ansprüche 2 bis 8.

So kann die Ratsche einstückig ausgebildet sein. Hierdurch ist es vorteilhaft möglich, die Herstellungskosten der Ratsche erheblich zu minimieren, da sich neben einer Montage auch ein Zusammenbau, wie etwa bei mehrteiligen Ratschen, erübrigt. Darüber hinaus ermöglicht die einstückige Ausbildung der Ratsche ein deutlich geringeres Risiko von anhaftenden Blut- oder Geweberesten und die daraus resultierende Infektionsgefahr durch Bakterienbildung. Auch die nach jeder Anwendung notwendige Sterilisation kann deutlich schneller durchgeführt werden, da die Ratsche nicht mehr zerlegt und anschließend neu zusammengebaut werden muss. Durch das Ermöglichen einer solchen effizienteren Arbeitsweise wird auch das stark beanspruchte Gesundheitssystem entlastet. Aufgrund der einstückigen Gestaltung der Ratsche kann ferner auf vorteilhafte Weise verhindert werden, dass bei mehreren baugleichen bzw. bauähnlichen Ratschen die Komponenten beim Zusammensetzen verwechselt werden, was unter Umständen zu einer Zerstörung oder einer Beeinträchtigung der Funktionsweise der Ratsche führen kann. Gerade bei einem wiederholten Zusammensetzen der Ratsche werden zudem die filigranen Verbindungsstellen der Bauteile stark in Anspruch genommen und verschließen. Mit einer einstückigen Ausbildung wird diesem Problem wirksam Rechnung getragen.

Gemäß einer weiteren Ausführungsform kann die Spirale der Ratsche einen Winkel von wenigstens 360 °, vorzugsweise von wenigstens 450 °, besonders bevorzugt von wenigstens 630 ° aufweisen. Der Winkel erstreckt sich dabei von dem Übergang von dem Griffstück zur Spirale bis hin zu einem freien Ende der Spirale. Die Gestaltung des Winkelmaßes bzw. der Umdrehungen der Spirale hängt von mehreren Faktoren ab. So kann z.B. die Rauheit der Oberfläche, welche in Wirkverbindung mit einer Manteloberfläche des Eindrehwerkzeugs steht, das Winkelmaß bestimmten. Je höher die Rauheit ist, desto weniger Winkelmaß der Spirale ist notwendig. Auch die Biegeelastizität der Spirale verändert die Wahl des Winkelmaßes, da durch sie bestimmt wird, in welchem Maße die Spirale das Eindrehwerkzeug umschließt. Es sei angemerkt, dass die Biegeelastizität der Spirale von deren Querschnitt abhängig ist. Da sich die Spannungsverteilung über die gesamte Bogenlänge der Spirale verteilt, ist die Spannungsverteilung besser, je größer das Winkelmaß ist. Indem die Spirale mit deren erster Umdrehung das Eindrehwerkzeug umgibt, ist es auch möglich das Griffstück unter einem Winkel anzuordnen. So wird die Zugänglichkeit der Ratsche zu einem beengten Raum, wie z.B. in der Kieferchirurgie, verbessert.

Gemäß einer weiteren Ausführungsform kann die Spirale wenigstens einen einer Drehachse zugewandten Vorsprung aufweisen. Der Vorsprung ist dabei derart an der Spirale ausgebildet, dass er mit einer korrespondierenden Ausnehmung eines Eindrehwerkzeugs in Eingriff stehen kann. So kann erreicht werden, dass bei Drehung der Ratsche in Drehmomentübertragungsrichtung zusätzlich zur zwischen dem das Eindrehwerkzeug umschließenden Abschnitt der Spirale und dem Eindrehwerkzeug vorliegenden kraftschlüssigen Verbindung (Reibung) unterstützend eine formschlüssige Verbindung bereitgestellt wird. Ferner können auch mehrere Vorsprünge entlang der Spirale ausgebildet sein, sodass die Kraft- bzw. Drehmomentübertragung von der Ratsche zum Eindrehwerkzeug im Vergleich zu lediglich einem Vorsprung gleichmäßiger auf das Eindrehwerkzeug verteilt werden kann. Das zu übertragende Drehmoment wird von der Steigung der Spirale in Verbindung mit dem Vorsprung beeinflusst. Es sei noch angemerkt, dass die korrespondierenden Ausnehmungen dementsprechend entlang dem Eindrehwerkzeug auszubilden sind, um eine gleichmäßige Drehmomentübertragung zu ermöglichen. Das zu übertragende Drehmoment ist von einer Vorsprungshöhe, vom Material der Ratsche und/oder dessen Härte abhängig.

Nach einer weiteren Ausführungsform kann der der Drehachse zugewandte Vorsprung an einem freien Ende der Spirale angeordnet sein. Die Anordnung des Vorsprungs am Ende der Spirale ermöglicht eine wirksame Kombination von kraftschlüssiger Verbindung und formschlüssiger Verbindung, da der das Eindrehwerkzeug umschließende Abschnitt die kraftschlüssige Verbindung sicherstellt und erst am Ende der Spirale der Formschluss erfolgt. So muss der Vorsprung gerade soviel Kraft- bzw. Drehmomentübertragung übernehmen, wie von der kraftschlüssigen Verbindung (Reibung) nicht erzielt werden konnte. Somit kann vorteilhaft der am Ende angeordnete Vorsprung entlastet werden, wodurch sein durch Kraftübertragung bedingter Verschleiß verringert werden kann.

Ferner kann eine dem freien Ende der Spirale abgewandte Fläche des Vorsprungs mit einer der Drehachse zugewandten Flanke einen Winkel ausbilden. Bei einer spitzwinkligen Ausbildung des Winkels kann erreicht werden, dass der Vorsprung fester in die Ausnehmungen des Eindrehwerkzeugs greift, also besser verhakt. und somit die gegenseitige Arretierung verbessert wird. Wird im Gegensatz dazu, der Winkel stumpf ausgebildet, kann auf vorteilhafte Weise je nach Bedarf eine Drehmomentbegrenzung realisiert werden, da sich der Vorsprung unter einen vorbestimmten Drehmoment von der Ausnehmung des Eindrehwerkzeugs löst. Je weiter der Winkel stumpf ausgebildet wird, desto niedriger ist das Drehmoment um den Vorsprung von dem Eindrehwerkzeug wegzubiegen. Der Winkel kann wahlweise zwischen 45 ° und 150 °, vorzugsweise zwischen 70 ° und 120 °, besonders bevorzugt zwischen 80 ° und 110 °, ausgebildet sein.

Darüber hinaus kann die Spirale eine über eine Länge erstreckende Ausnehmung aufweisen, wobei die Ausnehmung unmittelbar an die dem freien Ende der Spirale abgewandte Fläche des Vorsprungs angrenzt. Hierdurch wird das freie Ende der Spirale durch einen verringerten Querschnitt der Spirale in diesem Bereich biegelastischer als ohne Ausnehmung, was eine Drehmomentübertragungsbegrenzung ermöglicht, da der am Ende angeordnete Vorsprung sich ebenfalls von einem Eindrehwerkzeug wegbiegt. Somit lässt sich die Biegeelastizität am freien Ende je nach Bedarf einstellen. Die Ausnehmung kann sich ferner über die vollständige Dicke der Spirale erstrecken.

Gemäß einer weiteren Ausführungsform kann die der Drehachse zugewandte Flanke der Spirale wenigstens in einem Abschnitt aufgeraut sein. Mit einer derartigen Rauheit kann der Schlupf zwischen Ratsche und Eindrehwerkzeug minimiert oder vermieden werden, sodass auf vorteilhafte Weise erreicht wird, dass die vom Benutzer aufgebrachte Kraft im Wesentlichen ohne Schlupf auf das Eindrehwerkzeug übertragen wird. Generell kann die Rauheit in drei Bereiche eingeteilt werden. Bei dem ersten Bereich von Raₘᵢₙ bis Ra₁ liegt eine Art mechanische Adhäsion vor. Hierbei liegt ein durch eine molekulare Wechselwirkung in der Grenzflächenschicht hervorgerufener mechanischer Zusammenhalt der beteiligten Oberflächen vor. Die Oberflächen werden somit derart glatt ausgebildet, dass die vor dem Aufeinandertreffen vorhandene Luft entweicht und somit die beiden beteiligten Oberflächen fest aneinanderhaften. Bei dem zweiten Bereich von Ra₁ bis Ra₂ liegt Gleitreibung vor, welcher sich nicht für die Übertragung von Drehmoment eignet. Bei dem dritten Bereich zwischen Ra₂ bis Raₘₐₓ handelt es sich um Haftreibung, bei der die Oberflächenstruktur der beiden aufeinandertreffenden Oberflächen derart rau ausgebildet sind, dass sie haften. Ein weiterer, vierter Bereich, der größer als Raₘₐₓ ist, kann als Formschluss angesehen werden.

Bei der erfindungsgemäßen Ratsche kann als weiterer Aspekt die Ratsche aus Edelstahl, Titan, Keramik, verschleißfesten Kunststoffen oder dergleichen ausgebildet sein. Angesichts einer langen Lebensdauer der Ratsche wird verstärkt Wert auf die Verschleißfestigkeit bzw. Widerstandsfestigkeit der Ratsche, insbesondere bei den Klemmflächen gelegt. Ferner kann die Funktionsweise über einen langen Zeitraum gewährleistet werden. Alternativ können auch jede Art von Automaten-, Bau- oder Federstählen verwendet werden, die ein besonderes Beschichtungsverfahren durchgelaufen haben, um gegenüber Korrosion geschützt zu sein. Als besonders geeignet haben sich Federstahl 1.4310, Titan Grade 4, Titan Grade 5, PEEK erwiesen. Des Weiteren kommen sterilfähige Elastomere in Betracht. Es sei angemerkt, dass je nach Wahl des Werkstoffs die Biegeelastizität der Spirale eingestellt werden kann.

Als weiterer Aspekt kann die der Drehachse zugewandte Flanke der Spirale wenigstens in einem Abschnitt gehärtet ausgebildet sein. Wesentlich für den geringen Verschleiß der Ratsche ist, dass die Ratsche, insbesondere die der Drehachse zugewandte Flanke der Spirale, härter ausgebildet ist als das Eindrehwerkzeug. Dabei können unterschiedliche Härteverfahren, wie z.B. Umwandlungshärtung Kornfeinung, Ausscheidungshärtung, Mischkristallverfestigung, Kaltverfestigung, etc., zu Anwendung kommen. Des Weiteren ist es von Vorteil, wenn die Ratsche bezüglich des Härtegrads abgestimmt auf das zugehörige Eindrehwerkzeug ausgebildet ist, damit die Ratsche und das Eindrehwerkzeug ein korrelierendes Paar ergeben.

Gemäß einer weiteren Ausführungsform kann der wenigstens eine Vorsprung der Spirale wenigstens in einem Abschnitt gehärtet ausgebildet sein. So kann wirksam vermieden werden, dass sich der wenigstens eine Vorsprung abrundet und somit nicht mehr passgenau mit der wenigstens einen korrespondierenden Ausnehmung in Eingriff steht. Ferner kann ein durch Abrundung des Vorsprungs unerwünscht auftretender Schlupf vermieden werden.

Bei einer Ausführungsform der erfindungsgemäßen Ratsche kann im Bereich des Griffstücks und/oder des Drehpunktgehäuses bzw. der Spirale eine Markierung vorgesehen werden. Vorteilhaft ist die Markierung besonders für den Benutzer, wenn er an sensiblen Stellen arbeitet, wie z.B. am Gewebe, an Wunden, im Mundbereich, etc., da er durch die Markierung hingewiesen wird in welche Richtung die Ratsche gedreht werden muss, um das Eindrehwerkzeug anzuziehen oder wieder zu lösen. Demnach kann wirksam ein unnötiges "Ausprobieren" der Drehrichtung durch die Ratsche an sensiblen Stellen verhindert werden.

In einer weiteren Ausgestaltungsweise der erfindungsgemäßen Ratsche kann das Griffstück und das Drehpunktgehäuse die selbe Dicke aufweisen. Dadurch kann die Ratsche aus einer konstanten Materialdicke hergestellt werden, was die Herstellungskosten sinken lässt und den Fertigungsprozess wesentlich vereinfacht.

Gemäß einem weiteren Aspekt der vorliegenden Erfindung kann das Griffstück zylindrisch ausgebildet sein, wodurch dem Benutzer eine ergonomische Handhabung ermöglicht wird.

Nach einem weiteren Aspekt der erfindungsgemäßen Ratsche kann diese einen Anschlag aufweisen. Der Anschlag ragt wenigstens zum Teil in den Bereich des Drehpunktgehäuse rein, erstreckt sich vorzugsweise über eine parallele Ebene im Vergleich zu der Ebene, in der sich die Spirale eindreht, und ist auf der nicht zum Eindrehwerkzeug zugewandten Seite angeordnet, so dass die Ratsche beim Aufbringen auf das Eindrehwerkzeug lediglich bis zum Anschlag auf das Eindrehwerkzeug aufgebracht werden kann. Beim Aufbringen der Ratsche auf das Eindrehwerkzeug wird demnach wirksam verhindert, dass die Ratsche in axialer Richtung des Eindrehwerkzeugs in das Gewebe oder die offene Wunden gleitet, wodurch der Patient möglicherweise verletzt werden könnte.

Gemäß einem weiteren Aspekt der Erfindung wird nach Anspruch 9 ferner ein Drehmomentübertragungssystem geschaffen, das eine erfindungsgemäße Ratsche und ein Eindrehwerkzeug aufweist. Ferner weist das Eindrehwerkzeug einen ersten Abschnitt, der zylindrisch ausgebildet ist, und einen zweiten Abschnitt auf, der zylindrisch oder kegelstumpfartig ausgebildet ist, wobei der zweite Abschnitt ferner eine Aufnahme für ein medizinisches Werkzeug, chirurgisches Implantat oder einen anderen Gegenstand aufweist.

Mit Hilfe eines solchen Drehmomentübertragungssystems kann bei Drehung der Ratsche in Drehmomentübertragungsrichtung auf einfache Weise die Kraft eines Benutzers über die Ratsche zum Eindrehwerkzeug übertragen werden, ohne einen durch Schlupf bedingten Kraft- bzw. Drehmomentverlust.

Unter einem medizinischen Werkzeug kann ein Inbuskopf, Torxkopf, Fräskopf, Polierkopf, Pin-Aufnahmen, Pinzette, Feile oder dergleichen verstanden werden.

Vorteilhafte Weiterbildungen des erfindungsgemäßen Drehmomentübertragungssystems sind Gegenstand der abhängigen Ansprüche 10 bis 13.

So kann zwischen der Ratsche und dem Eindrehwerkzeug eine kraftschlüssige Verbindung vorliegen. Auf einfache Weise kann so unter Zuhilfenahme von Reibung die Drehmomentübertragung realisiert werden.

Alternativ oder ergänzend kann zwischen der Ratsche und dem Eindrehwerkzeug eine formschlüssige Verbindung vorliegen. Die Drehmomentübertragung von der Ratsche zum Eindrehwerkzeug kann so besonders vorteilhaft erfolgen.

Ferner kann der erste Abschnitt am Eindrehwerkzeug wenigstens eine entlang dessen Manteloberfläche angeordnete Ausnehmung aufweisen, wobei bei einer solchen formschlüssigen Verbindung erreicht wird, dass der wenigstens eine Vorsprung der Spirale mit der wenigstens einen Ausnehmung des Eindrehwerkzeugs in Eingriff steht. Demzufolge kann auf einfache Weise die formschlüssige Verbindung hergestellt werden.

Zudem kann entlang der Manteloberfläche des Eindrehwerkzeugs eine Mehrzahl der Ausnehmungen mit einem periodischen Abstand bzw. einer Teilung angeordnet sein. Somit wird besonders vorteilhaft die Kraftübertragung von der Spirale über wenigstens einen Vorsprung auf das Eindrehwerkzeug übertragen.

Gemäß einem weiteren Aspekt der Erfindung wird nach Anspruch 14 die Verwendung einer erfindungsgemäßen Ratsche im Medizinbereich, insbesondere in der Chirurgie oder im Dentalbereich, zum Übertragen eines Drehmoments auf ein Eindrehwerkzeug vorgeschlagen.

Gemäß noch einem weiteren Aspekt der vorliegenden Erfindung wird nach Anspruch 15 ein Verfahren zum Übertragen eines aufzubringenden Drehmoments auf ein Eindrehwerkzeug vermittels einer erfindungsgemäßen Ratsche geschaffen, mit den Schritten: Aufsetzen der Ratsche auf das Eindrehwerkzeug in dessen axialer Richtung, sowie vorzugsweise Drehen der Ratsche in eine Freilaufrichtung bei gleichzeitigem Absenken der Ratsche entlang des Eindrehwerkzeugs; Fixieren der Ratsche auf dem Eindrehwerkzeug und Übertragen des Drehmoments auf das Eindrehwerkzeug durch Drehen der Ratsche in eine Drehmomentübertragungsrichtung; Freigeben des Eindrehwerkzeugs durch Drehen der darauf befindlichen Ratsche in Freilaufrichtung; und Lösen der Ratsche vom Eindrehwerkzeug durch Drehen in Freilaufrichtung mit gleichzeitigem Abnehmen der Ratsche vom Eindrehwerkzeug in dessen axialer Richtung.

Damit werden die oben anhand der erfindungsgemäßen Ratsche erläuterten Vorteile und Effekte gleichermaßen erzielt.

Vorteilhafte Weiterbildungen des erfindungsgemäßen Verfahrens sind Gegenstand der abhängigen Ansprüche 16 bis 18.

Gemäß einer Ausführungsform des erfindungsgemäßen Verfahrens werden die Schritte Fixieren der Ratsche auf dem Eindrehwerkzeug und Übertragen des Drehmoments auf das Eindrehwerkzeug durch Drehen der Ratsche in Drehmomentübertragungsrichtung; sowie Freigeben des Eindrehwerkzeug durch Drehen der darauf befindlichen Ratsche in Freilaufrichtung bevorzugt wiederholt ausgeführt, bevor die Ratsche vom Eindrehwerkzeug gelöst wird.

Ferner kann der Schritt des Fixierens der Ratsche auf dem Eindrehwerkzeug und die Übertragung des Drehmoments auf das Eindrehwerkzeug durch Drehen der Ratsche in Drehmomentüberträgungsrichtung so ausgeführt werden, dass wenigstens ein Vorsprung einer Spirale mit wenigstens einer Ausnehmung des Eindrehwerkzeugs in Eingriff steht; und der Schritt Freigeben des Eindrehwerkzeugs durch Drehen der darauf befindlichen Ratsche in Freilaufrichtung so ausgeführt werden, dass sich der wenigstens eine Vorsprung der Spirale von der wenigstens einen Ausnehmung des Eindrehwerkzeugs löst.

Gemäß einer weiteren bevorzugten Ausführungsform des erfindungsgemäßen Verfahrens wird der Schritt Fixieren der Ratsche auf dem Eindrehwerkzeug und Übertragen des Drehmoments auf das Eindrehwerkzeug durch Drehen der Ratsche in Drehmomentübertragungsrichtung so ausgeführt, dass ein erster Teil des an dem Drehpunktgehäuse aufgebrachten Drehmoments die biegeelastische Spirale weiter in eine Ebene eindreht, so dass zwischen einer der Drehachse zugewandten Flanke und dem Eindrehwerkzeug eine kraftschlüssige Verbindung vorliegt, und dass ein zweiter Teil des an dem Drehpunktgehäuse aufgebrachten Drehmoments eine Drehbewegung des Eindrehwerkzeugs verursacht; und der Schritt Freigeben des Eindrehwerkzeugs durch Drehen der darauf befindlichen Ratsche in Freilaufrichtung so ausgeführt, dass sich die kraftschlüssige Verbindung zwischen der der Drehachse zugewandten Flanke der Spirale und dem Eindrehwerkzeug löst.

Es sei angemerkt, dass die beiden Schritte Fixieren der Ratsche sowie Freigeben des Eindrehwerkzeugs bei der vorstehenden Ausführungsform jeweils mit den entsprechenden Schritten dieser Ausführungsform kombiniert werden können.

Mit dem erfindungsgemäßen Verfahren sowie der erfindungsgemäßen Verwendung der Ratsche lassen sich dabei in analoger Weise die gleichen Vorteile erzielen wie vorstehend in Zusammenhang mit der Ratsche diskutiert. Zur Vermeidung von Wiederholungen wird daher auf eine erneute Aufzählung derselben verzichtet.

Gemäß noch einem weiteren Aspekt der vorliegenden Erfindung wird nach Anspruch 19 ein Verfahren zum Herstellen einer Ratsche geschaffen, mit den Schritten: Auswählen eines Rohlings; und Ausbilden einer Kontur der Ratsche vermittels Laserschneiden, Wasserstrahlschneiden, Drahterodieren, Funkenerodieren oder Stanzen.

Auf diese Weise kann die erfindungsgemäße Ratsche besonders einfach, präzise und mit geringen Kosten gefertigt werden.

Eine beispielhafte vorteilhafte Weiterbildung des erfindungsgemäßen Verfahrens ist Gegenstand des abhängigen Anspruchs 20.

Gemäß dieser bevorzugten Ausführungsform des Verfahrens wird der Rohling derart gewählt, dass er eine einheitliche Dicke aufweist. Durch diese vorteilhafte Ausgestaltung kann die Ratsche aus einer konstanten Materialdicke hergestellt werden, was die Herstellungskosten sinken lässt und den Fertigungsprozess wesentlich vereinfacht.

Die vorstehend beschriebenen Merkmale und Funktionen der vorliegenden Erfindung sowie weitere Aspekte und Merkmale werden nachfolgend anhand einer detaillierten Beschreibung von bevorzugten Ausführungsformen unter Bezugnahme auf die beigefügten Figuren weiter beschrieben. Hierbei zeigt:
- Fig. 1: eine Draufsicht einer erfindungsgemäßen Ratsche;
- Fig. 2: eine Seitenansicht der erfindungsgemäßen Ratsche gemäß Fig.1;
- Fig. 3: eine Draufsicht einer erfindungsgemäßen Ratsche mit einem Vorsprung an einem freien Ende einer Spirale;
- Fig. 4: eine Draufsicht einer erfindungsgemäßen Ratsche mit einer eineinhalb Umdrehungen aufweisenden Spirale;
- Fig. 5: einen Querschnitt einer erfindungsgemäßen Ratsche mit einem Anschlag;
- Fig. 6 a)-f): mehrere vergrößerte Darstellungen des Vorsprungs an der erfindungsgemäßen Ratsche;
- Fig. 7: eine Draufsicht einer erfindungsgemäßen Ratsche mit einem Vorsprung an einem freien Ende und einer angrenzende Ausnehmung einer Spirale;
- Fig. 8a: eine Seitenansicht eines Eindrehwerkzeugs;
- Fig. 8b: eine Draufsicht des Eindrehwerkzeugs gemäß Fig. 7a; und
- Fig. 9: eine Seitenansicht eines weiteren Eindrehwerkzeugs.

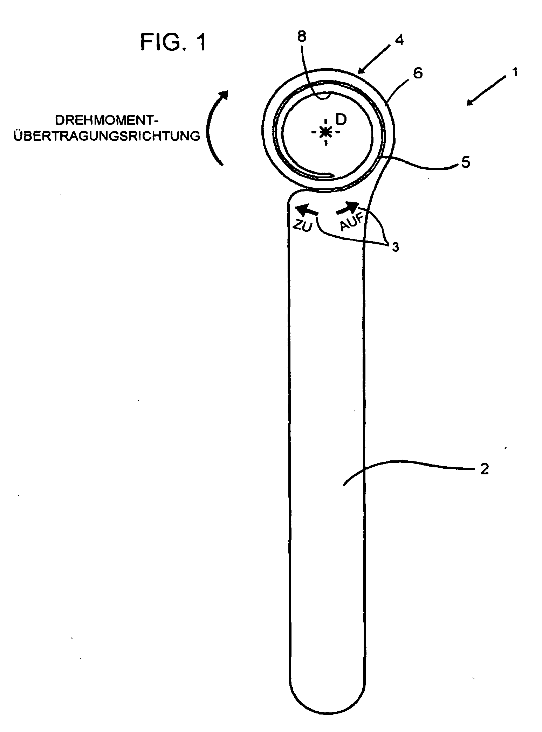

Fig. 1 stellt eine Draufsicht einer Ratsche 1 gemäß einer ersten beispielhaften Ausführungsform der vorliegenden Erfindung dar.

Die Ratsche 1 gemäß der ersten Ausführungsform ist einstückig ausgebildet und besteht aus einem Griffstück 2 und einem Drehpunktgehäuse 4, welche die gleiche Dicke d aufweisen. Das Drehpunktgehäuse 4 ist als archimedische Spirale 6 mit zwei Umdrehungen ausgebildet. In dem Drehpunktgehäuse 4 befindet sich eine Drehachse D, bezüglich der die Drehmomentenübertragung von der Ratsche 1 zu einem Eindrehwerkzeug 100 ausgeführt wird. Es sei noch angemerkt, dass sich der Pfeil mit der eingezeichneten Drehmomentübertragungsrichtung auf die Drehung der Ratsche 1 bezieht.

Da die Spirale 6 mit zwei Umdrehungen ausgebildet ist, erhöht sich die Anpresskraft auf das Eindrehwerkzeug 100 auch dadurch, dass bei Drehung der Ratsche 1 in Drehmomentübertragungsrichtung ein bei einer neutralen Stellung der Ratsche 1 bzw. bei der Freilaufrichtung vorhandener Zwischenraum 5 nicht mehr vorhanden ist, und somit aufeinandertreffende Flanken der Spirale die Drehmomentübertragung wesentlich unterstützen. Diese Flanken sind auch aufgeraut, um die Drehmomentübertragung zu unterstützen.

Eine der Drehachse D zugewandte Flanke 8 der Spirale 6 ist aufgeraut, sodass die Drehmomentübertragung von der Spirale 1 auf das Eindrehwerkzeug 100 wirksamer erfolgen kann. Ferner ist die der Drehachse D zugewandte Flanke 8 gehärtet, um den Verschleiß gegenüber dem Eindrehwerkzeug 100 zu minimieren oder zu vermeiden.

Markierungen 3 auf der Ratsche 1, hier im Bereich des Griffstücks 2 aufgebracht, die eine Öffnen-/Schließrichtung anzeigen, dienen dem Benutzer für eine einfache Handhabung der Ratsche 1, so dass Verletzungen am Gewebe oder im Mundbereich wirksam vermieden werden können. Hiermit ist es für den Benutzer klar ersichtlich, in welche Richtung er die Ratsche 1 für eine Drehmomentübertragung respektive Freilauf betätigen muss. Die auf der Ratsche 1 befindlichen Markierungen 3 können auch aufgeklebt, geätzt, graviert, gelasert oder durch eine andere Hoch-, Tief-, Durch-, Tampon-, Sieb- oder Flachdrucktechnik ausgebildet werden.

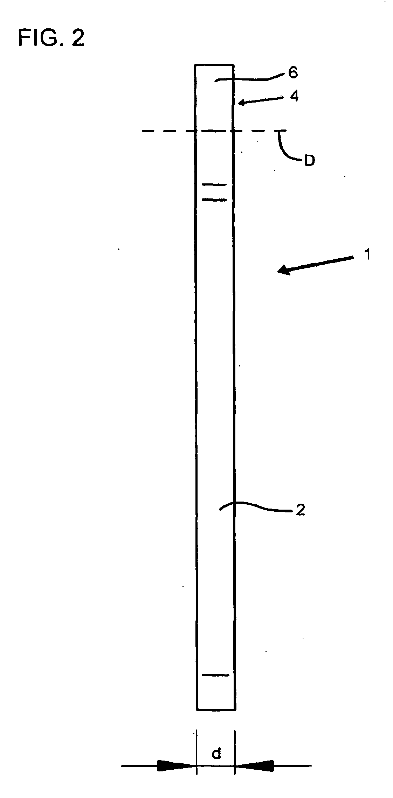

In Fig. 2 ist eine Seitenansicht der Ratsche 1 gemäß Fig.1 dargestellt, in der erkennbar ist, dass sich die Spirale 6 der Ratsche 1 in einer Ebene, also zweidimensional eindreht. Die Dicke d der Ratsche 1 beträgt hierbei 2 mm.

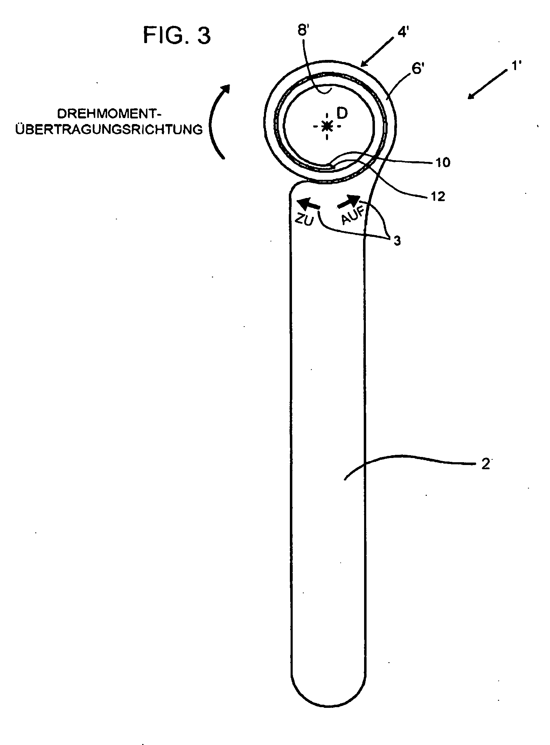

Fig. 3 stellt eine Draufsicht einer Ratsche 1' mit einem Vorsprung 10 in einer zweiten beispielhaften Ausführungsform der vorliegenden Erfindung dar. Der Vorsprung 10 ist dabei an einem freien Ende 12 einer Spirale 6' an einer der Drehachse D zugewandten Flanke 8' der Spirale 6' angeordnet.

Die Ratsche 1' gemäß der zweiten Ausführungsform ist ferner einstückig ausgebildet und besteht ebenfalls aus einem Griffstück 2 und einem Drehpunktgehäuse 4', welche die gleiche Dicke d aufweisen. Die Drehachse D befindet sich innerhalb des Drehpunktgehäuses 4'. In gleicher Weise wie in Fig. 1 bezieht sich der Pfeil mit der eingezeichneten Drehmomentübertragungsrichtung auf die Drehung der Ratsche 1'.

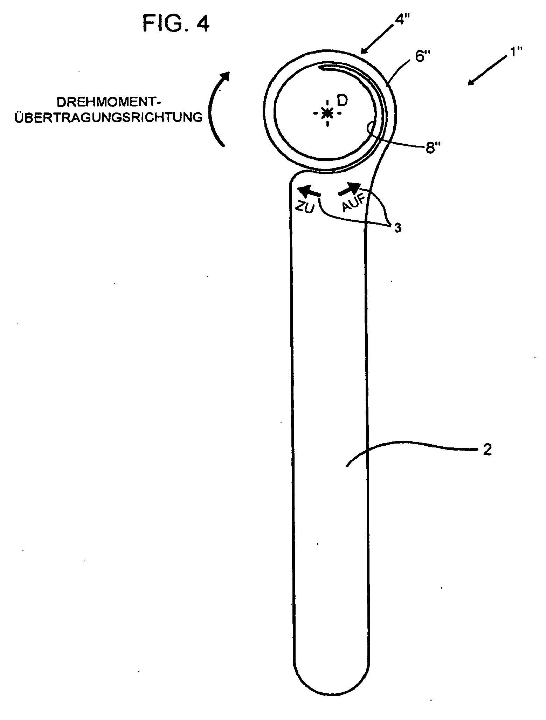

Fig. 4 stellt eine Draufsicht einer Ratsche 1 " gemäß einer dritten Ausführungsform mit einer eineinhalb Umdrehungen aufweisenden Spirale 6" dar.

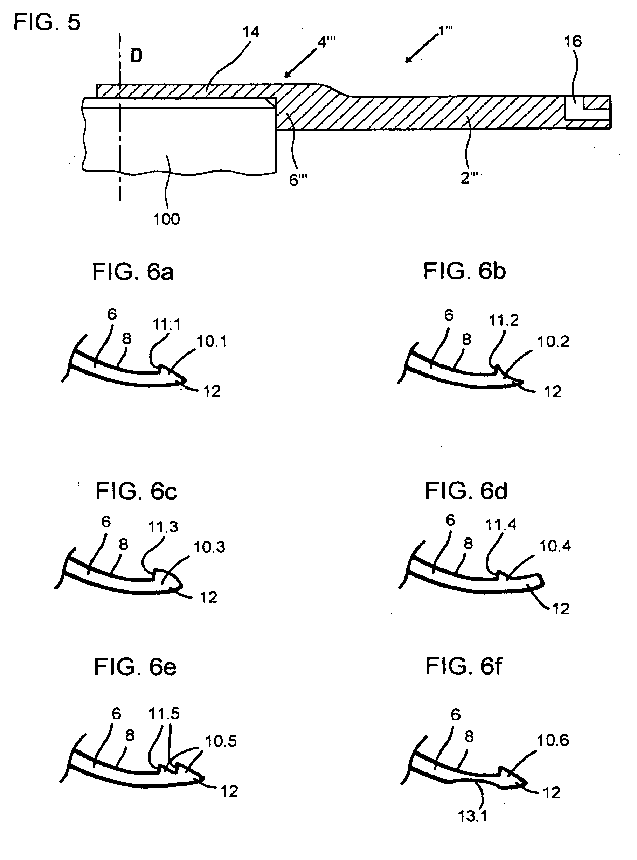

Fig. 5 stellt einen Querschnitt einer Ratsche 1"' mit einem Anschlag 14 nach einer weiteren Ausführungsform der vorliegenden Erfindung dar. Der aus dem Drehpunktgehäuse 4"' und/oder dem Griffstück 2"' herausragende Anschlag 14 ist derart ausgebildet, dass er wenigstens zum Teil in den Bereich einer Eindrehwerkzeugaufnahme ragt, so dass die Ratsche 1"' lediglich bis zu einem im Voraus definierten Bereich auf ein Eindrehwerkzeug 100 aufgebracht werden kann. Damit können Verletzungen am Gewebe durch zu "tiefes" Einsetzen wirksam vermieden werden. Das Griffstück 2"' weist ferner eine Aufnahme 16 auf der dem Drehpunktgehäuse 4"' abgewandten Seite auf. Diese Aufnahme 16 kann zum Anbringung einer Verlängerung des Griffstücks 2"' dienen, um beispielsweise Stellen zu erreichen, die sonst unzugänglich wären.

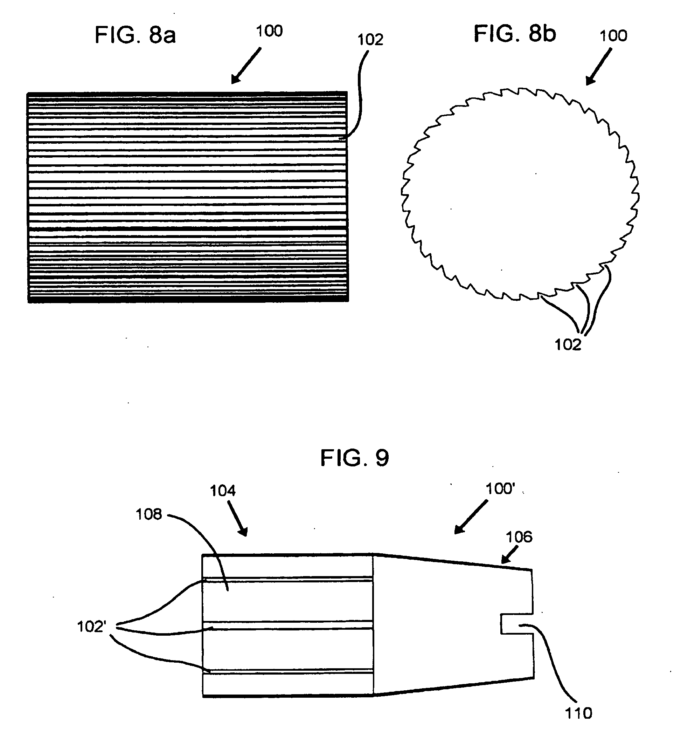

Fig. 6 stellt mehrere vergrößerte Darstellungen des Vorsprungs 10 an der Spirale 6' gemäß der Ratsche 1' dar. Bei der Darstellung 6a) ist ein Vorsprung 10.1 am Ende 12 der Spirale 6' keilartig ausgebildet. Der Vorsprung 10.1 ist dabei derart keilförmig ausgebildet, dass der Keil in Richtung dem Ende 12 abfällt. Durch diesen keilartigen Vorsprung 10.1 kann ein in Fig. 8a und 8b gezeigtes Eindrehwerkzeug 100 in Drehmomentübertragungsrichtung fest arretiert und in Freilaufrichtung durch die Keilform besonders gut wieder freigegeben werden. In der Darstellung 6b) ist ein Vorsprung 10.2 ebenso am Ende 12 positioniert. Der Vorsprung 10.2 weist dabei eine dem Ende 12 abgewandte orthogonale Fläche 11.2 bezüglich der Flanke 8 auf und auf der dem Ende 12 zugewandten Fläche eine konkave Ausbildung auf. Anstatt der konkaven Ausbildung ist in Darstellung 6c) ein Vorsprung 10.3 konvex ausgebildet. Die Darstellung 6d) verdeutlicht, dass die Anordnung eines Vorsprungs 10.4 auch entlang der der Drehachse D zugewandten Flanke 8 erfolgen kann. Auf gleiche Weise können auch mehrere Vorsprünge 10.5, wie aus der Darstellung 6e) verdeutlicht wird, an der der Drehachse D zugewandten Flanke 8 angeordnet sein. Die Anzahl der Vorsprünge 10.5 ist hierbei nicht auf zwei, wie in Darstellung e) gezeigt, begrenzt. Ferner können die Vorsprünge 10.5 auch mit einem vorbestimmten Abstand angeordnet sein. Bei der Darstellung 6b) ist eine Ausbildung wie in Darstellung 6a) dargstellt mit dem Unterschied, dass die Spirale 6 auf der abgewandten Fläche der Drehachse D eine Ausnehmung 13.1 aufweist. Diese Ausnehmung 13.1 erstreckt sich ab dem Vorsprung 10.6 über eine vorbestimmte Länge. Auf diese Weise kann die Biegelastizität des Bereichs des Vorsprungs eingestellt werden.

Durch diese vorstehenden Ausgestaltungen des Vorsprungs/der Vorsprünge 10 kann eine formschlüssige Verbindung zwischen der Ratsche 1 und dem Eindrehwerkzeug 100 erzielt werden.

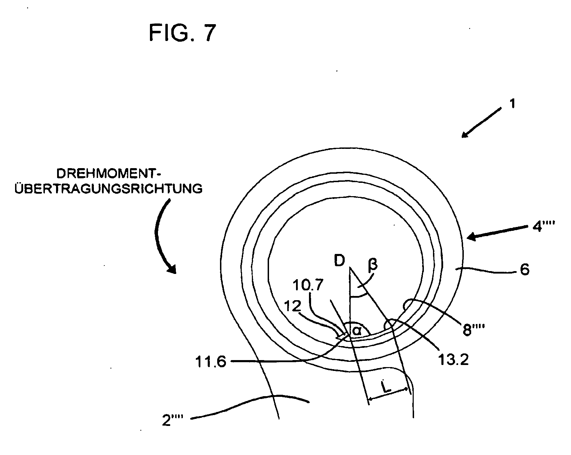

Fig. 7 stellt eine Draufsicht einer erfindungsgemäßen Ratsche 1"" mit einem Vorsprung 10.7 an einem freien Ende 12 und einer angrenzende Ausnehmung 13.2 einer Spirale 6"" dar.

Die Ratsche gemäß dieser vierten Ausführungsform ist aus einem Griffstück 2"" und einem Drehpunktgehäuse 4"" ausgebildet, welche beide eine gleiche Dicke d aufweisen. Das Drehpunktgehäuse 4"" ist als Spirale 6"" ausgebildet, die an deren freien Ende 12 einen keilförmigen Vorsprung 10.7 aufweist. Der Vorsprung 10.7 ist dabei derart keilförmig ausgebildet, dass der Keil in Richtung dem freien Ende 12 der Spirale 6"" abfällt. Ferner weist die Spirale 6"" eine über eine Länge L erstreckende Ausnehmung 13.2 auf. Ferner weist der Vorsprung 10.7 eine dem freien Ende 12 der Spirale 6"" abgewandte Fläche 11.6 auf, die durch einen Winkel α definiert ist. Der Winkel α erstreckt sich dabei von der dem freien Ende 12 abgewandten Fläche 11.6 bis zu einer der Drehachse D zugewandten Flanke 8.

Die der Drehachse D zugewandte Flanke 8 ist unmittelbar von der dem freien Ende 12 abgewandten Fläche mit einer Ausnehmung 13.2 versehen, die sich über eine vorbestimmte Länge L erstreckt. Diese Ausnehmung 13.2 grenzt unmittelbar an die dem freien Ende 12 der Spirale 6"" abgewandte Fläche 11.6 des Vorsprungs 10.7 an. Der Übergang zwischen einer kontinuierlichen Verjüngung und der Ausnehmung 13.2 ist kontinuierlich ausgebildet.

Obgleich die Länge der Ausnehmung 13.2 der Spirale 6"" durch eine Länge L gemessen wird, kann sie ohne Weiteres auch in Bogenmaß mit dem Winkel β gemessen werden, wie aus der Fig. 7 ersichtlich. Ebenfalls ist es nicht erforderlich, dass die Ausnehmung 13.2 der Spirale 6"" unmittelbar an die dem freien Ende 12 abgewandte Fläche 11.6 angrenzt.

Ferner ist in Fig. 7 ersichtlich, dass sich der Querschnitt der Spirale 6"" selbst von dem am Griffstück 2"" angeordneten Ende bis hin zum freien Ende 12 verjüngt. Die zusätzliche Ausbildung der Ausnehmung 13.2 im Bereich des freien Endes 12, was als weitere zusätzliche Verjüngung zu verstehen ist, führt dazu, dass sich die Spirale 6"" im Bereich der Ausnehmung 13.2, und somit auch der Vorsprung 10.7, bei einer zu hohen Drehmomentübertragung biegeelastisch zurück biegt, mit anderen Worten von dem Eindrehwerkzeug 100 wegbiegt. Somit kann je nach Ausgestaltung der Ausnehmung 13.2 bzw. der Spirale 6"" in diesem Bereich eine Drehmomentbegrenzung vorgesehen werden.

Fig. 8a stellt eine Seitenansicht eines Eindrehwerkzeugs 100 dar. Fig. 8b ist eine Draufsicht eines Eindrehwerkzeugs 100, bei der eine Mehrzahl von Ausnehmungen 102 eine Teilung von 40 aufweist. Die Ausnehmungen 102 sind keilartig ausgebildet, mit anderen Worten komplementär zu dem Vorsprung 10 der Ratsche 1' gemäß Fig. 3. Vorteilhaft kann es jedoch auch sein, wenn eine Ratsche 1"" gemäß der vierten Ausführungsform zur Drehmomentübertragung verwendet wird, da dabei eine das Drehmoment je nach Bedarf begrenzt bzw. eingestellt werden kann. Das Eindrehwerkzeug 100 ist dabei zylindrisch ausgebildet und weist auf einer Manteloberfläche 108 eine Mehrzahl von Ausnehmungen 102 auf. Die Steigung der keilartigen Ausnehmungen 102 können unter Berücksichtigung der Ratschen ausgestaltet werden, sodass eine gewünschte Drehmomentübertragung eingestellt werden kann.

Fig. 9 stellt ein weiteres Eindrehwerkzeug 100' dar, das in zwei Abschnitte 104 und 106 unterteilt ist. Der erste Abschnitt 104 ist zylindrisch ausgebildet. Entlang von dessen Manteloberfläche 108 ist eine Mehrzahl von Ausnehmungen 102' angeordnet. Der zweite Abschnitt 106 ist kegelstumpfartig ausgebildet und weist an dessen Ende eine hier schematisch angedeutete Aufnahme 110 für ein medizinisches Werkzeug auf.

Zur Ausgestaltung des Griffstücks 2 sowie dessen Anzahl wird sowohl auf die Figuren 5a, 5b, 5c und 7a, 7b, 7c als auch auf die zugehörige Beschreibung der deutschen Patentanmeldung DE 10 2011 052 422 verwiesen.

Die Erfindung lässt neben den erläuterten Ausführungsformen weitere Gestaltungsansätze zu.

Die Drehachse des Drehgehäuses und eine Längsachse des Griffstücks können voneinander beabstandet sein. Das Drehpunktgehäuse kann somit zu dem Griffstück als Winkel ausgebildet werden. Mit dieser vorteilhaften Gestaltung der Ratsche kann der Benutzer vermittels der Beabstandung der Drehachse zu der Längsachse an schwer zugänglichen Stellen, insbesondere im Bereich der Backzähne, arbeiten. Die Drehachse des Drehgehäuses und die Längsachse des Griffstücks können sich auch schneiden.

Die Drehachse des Drehgehäuses und die Längsachse des Griffstücks können sich in einem Winkel von 30 ° bis 150 °, vorzugsweise von 60 ° bis 120 °, besonders bevorzugt von 90 °, relativ zur Längsachse schneiden. Vorteile bringt diese Ausgestaltung beim Aufbringen der Ratsche auf das Eindrehwerkzeug, insbesondere bei schwer zugänglichen Bereichen. Ohne Weiteres kann hierbei ein feststellbares Gelenk zwischen dem Drehpunktgehäuse und dem Griffstück angeordnet werden, dass eine kontinuierliche Einstellung des Winkels, je nach Bedarf, ermöglicht. Je größer das Winkelmaß (Bogenlänge) die Spirale aufweist, je mehr Neigung zum Schlüssel wird ermöglicht.

Zwischen dem Griffstück 2 und dem Drehpunktgehäuse 4 kann z.B. ein federartiger, zumindest senkrecht zur Drehachse D teilelastischer bzw. biegsamer, Übergang ausgebildet sein. Die Ausbildung des Übergangs kann mäanderförmig erfolgen. Vorteilhaft ist diese Ausbildung für das Aufsetzen der einstückig ausgebildeten Ratsche 1 auf das Eindrehwerkzeug 100.

Zwar ist die Ratsche in den vorstehenden Ausführungsformen einstückig ausgebildet, allerdings ist sie auch mehrstückig ausbildbar.

Ferner ist es möglich, dass eine einer Drehachse zugewandte Flanke auch glatt ausgebildet ist und die Drehmomentübertragung lediglich von wenigstens einem Vorsprung oder der durch die zusammengedrückte Spirale bedingten Federkraft erfolgt.

Die Dicke d der Ratsche 1 kann wahlweise je nach Baugröße zwischen 0,5 mm und 5 mm, vorzugsweise zwischen 1 mm und 4 mm, besonders bevorzugt 2,5 mm ausgebildet sein.

Zur besseren Handhabung kann das Griffstück geriffelt oder strukturiert ausgebildet sein. Obschon das Griffstück zylindrisch ausgebildet ist, kann es im Querschnitt auch dreieckig, quadratisch, rechteckig oder dergleichen sein.

Obwohl in der Spirale wenigstens ein Vorsprung ausgebildet werden kann, ist es ebenfalls möglich, wenigstens eine entsprechende punktuelle Ausnehmung vorzusehen und das Eindrehwerkzeug mit korrespondierenden Vorsprüngen vorzusehen. Ferner ist es auch denkbar, entlang der Spirale Ausnehmungen und Vorsprünge zu kombinieren.

Obgleich sich die Spirale in eine Ebene eindreht, kann sich eine weitergebildete Spirale über mehrere Ebenen (dreidimensional) eindrehen, sodass die eine der Drehachse zugewandte Flanke der Spirale das Eindrehwerkzeug mit einem Umschließungswinkel von mehr als 360 ° umschließt.

Obschon sich die Ausnehmungen mit einem periodischen Abstand entlang der Manteloberfläche erstrecken, können die Ausnehmungen auch aperiodisch ausgebildet sein.

Obwohl der Vorsprung gehärtet ausgebildet ist, kann er auch weichgeglüht ausgebildet sein.

Die Ausnehmung 13.2 muss nicht auf der der Drehachse D zugewandten Seite der Spirale vorliegen, sondern kann alternativ oder ergänzend auch auf der von der Drehachse D wegweisenden Seite bzw. Fläche der Spirale angeordnet sein. In einer vereinfachten Ausgestaltungsweise kann auf diese Ausnehmung 13.2 jedoch auch verzichtet werden.

Das zu übertragende Drehmoment kann beispielsweise auch von der Vorsprungshöhe in Verbindung mit der von der Drehachse wegweisenden Ausnehmungstiefe abhängen. Während einer Drehmomentübertragung kann diese Ausnehmung dem angrenzenden Bereich bzw. Abschnitt der Spirale bis hin zum Vorsprung und gegebenenfalls dem Vorsprung selbst einen ausreichenden Spielraum ermöglichen, um bei einer Übertragung eines möglicherweise zu großen Drehmoments zurückzufedern, weg vom Eindrehwerkzeug, und dadurch eine Drehmomentbegrenzung ermöglichen.

Obgleich die Vorsprünge an der der Drehachse zugewandten Fläche ausgebildet sind, können sie auch auf der Stirnseite der Ratsche ausgebildet sein. Somit kann die Ratsche bei beengtem Raum flexibler eingesetzt werden.

Ein alternatives Verfahren zur Herstellung der Ratsche kann derart durchgeführt werden, dass zunächst ein Rohling in Form eines Materialblocks ausgesucht wird. Dieser kann im Anschluss so bearbeitet werden, dass die gewünschte Kontur der Ratsche ausgebildet wird. Abschließend wird der die Ratschenkontur aufweisende Materialblock in einzelne Ratschen mit einer Dicke d abgetrennt. Wahlweise können die abgetrennten Ratschen weiterbehandelt werden. Hierbei können Laserschneiden, Wasserstrahlschneiden, Drahterodieren, Funkenerodieren oder Stanzen zum Einsatz kommen.

Obschon die Ratsche 1 vorstehend für den Medizinbereich, insbesondere für Dentalbereich, diskutiert wird, kann die Ratsche 1 auch in Bereichen, wo ein hoher Anspruch an Reinheit besteht, wie z.B. bei der Raumfahrt, bei der Halbleiterfertigung, etc., oder dergleichen eingesetzt werden.

Es wird eine Ratsche, insbesondere eine Ratsche für chirurgische Eingriffe oder eine Dentalratsche, geschaffen, die ein Griffstück und ein daran angeordnetes Drehpunktgehäuse, das als eine ebene Spirale ausgebildet ist, aufweist. Dabei ist die Spirale biegeelastisch ausgebildet und derart betätigbar, dass die Spirale sich in einer im Wesentlichen senkrecht zur Drehachse D orientierten Ebene eindreht. Ferner wird ein Drehmomentübertragungssystem vorgeschlagen, das eine Ratsche und ein Eindrehwerkzeug aufweist. Das Eindrehwerkzeug weist einen ersten Abschnitt, der zylindrisch ausgebildet ist, und einen zweiten Abschnitt auf, der zylindrisch oder kegelstumpfartig ausgebildet ist, wobei der zweite Abschnitt ferner eine Aufnahme für ein medizinisches Werkzeug, ein chirurgisches Implantat oder einen anderen Gegenstand aufweist. Damit wird eine verbesserte Ratsche erzielt, mit der das aufzubringende Drehmoment dosiert auf das Eindrehwerkzeug aufbringbar ist.

## Patentansprüche

1. Ratsche (1; 1'; 1 "; 1"') für den Medizinbereich, aufweisend:
ein Griffstück (2; 2'; 2"; 2"'), und
ein daran angeordnetes Drehpunktgehäuse (4; 4'; 4"; 4"'),
**dadurch gekennzeichnet, dass**
das Drehpunktgehäuse (4; 4'; 4"; 4"') als eine ebene Spirale (6; 6'; 6"; 6"') ausgebildet ist,
wobei die Spirale (6; 6'; 6"; 6"') biegeelastisch ausgebildet ist,
wobei die Ratsche (1; 1'; 1"; 1"') derart betätigbar ist, dass sich die Spirale (6; 6'; 6"; 6"') in einer im Wesentlichen senkrecht zur Drehachse (D) orientierten Ebene eindreht, und wobei
die Spirale (6; 6'; 6"; 6"') als eine logarithmische Spirale, eine hyperbolische Spirale oder eine archimedische Spirale ausgebildet ist.

2. Ratsche nach Anspruch 1, **dadurch gekennzeichnet, dass** sie einstückig, vorzugsweise aus Edelstahl, Titan, Keramik, verschleißfesten Kunststoffen oder dergleichen, ausgebildet ist.

3. Ratsche nach Anspruch 1 oder 2, **dadurch gekennzeichnet, dass** die Spirale (6; 6'; 6"; 6"') einen Winkel von wenigstens 360 °, vorzugsweise von wenigstens 450 °, besonders bevorzugt von wenigstens 630 ° aufweist.

4. Ratsche nach einem der Ansprüche 1 bis 3, **dadurch gekennzeichnet, dass** die Spirale (6; 6'; 6"; 6"') wenigstens einen der Drehachse (D) zugewandten Vorsprung (10; 10.1; 10.2; 10.3; 10.4; 10.5; 10.6; 10.7) aufweist, wobei der der Drehachse (D) zugewandte Vorsprung (10; 10.1; 10.2; 10.3; 10.6; 10.7) vorzugsweise an einem freien Ende (12) der Spirale (6; 6'; 6"; 6"') angeordnet ist, und wobei eine dem freien Ende (12) der Spirale (6"") abgewandte Fläche (11.6) des Vorsprungs (10.6) mit einer der Drehachse (D) zugewandten Flanke (8; 8') vorzugsweise einen Winkel (α) ausbildet.

5. Ratsche nach Anspruch 4, **dadurch gekennzeichnet, dass** die Spirale (6; 6'; 6"; 6"') eine sich über eine Länge (L) erstreckende Ausnehmung (13.2) aufweist, wobei die Ausnehmung (13.2) unmittelbar an die dem freien Ende (12 der Spirale (6"") abgewandte Fläche (11.6) des Vorsprungs (10.6) angrenzt.

6. Ratsche nach einem der Ansprüche 1 bis 5, **dadurch gekennzeichnet, dass** die der Drehachse (D) zugewandte Flanke (8; 8') der Spirale (6; 6'; 6"; 6"') wenigstens in einem Abschnitt aufgeraut ist, und/oder dass die der Drehachse (D) zugewandte Flanke (8; 8') der Spirale (6; 6'; 6"; 6"') wenigstens in einem Abschnitt gehärtet ausgebildet ist und/oder der wenigstens eine Vorsprung (10; 10.1; 10.2; 10.3; 10.4; 10.5; 10.6; 10.7) wenigstens in einem Abschnitt gehärtet ausgebildet ist.

7. Ratsche nach einem der Ansprüche 1 bis 6, **dadurch gekennzeichnet, dass** im Bereich des Griffstücks (2; 2'; 2"; 2"') und/oder des Drehpunktgehäuses (4; 4'; 4"; 4"') eine Markierung (3) vorgesehen ist, wobei das Griffstück (2; 2'; 2"; 2"') und das Drehpunktgehäuse (4; 4'; 4"; 4"') vorzugsweise die selbe Dicke (d) aufweisen und/oder das Griffstück (2; 2'; 2"; 2"') vorzugsweise zylindrisch ausgebildet ist.

8. Ratsche nach einem der Ansprüche 1 bis 7, **dadurch gekennzeichnet, dass** sie einen Anschlag (14) aufweist, der wenigstens zum Teil in den Bereich des Spiralzentrums ragt.

9. Drehmomentübertragungssystem, aufweisend:
eine Ratsche (1; 1'; 1"; 1"') nach wenigstens einem der Ansprüche 1 bis 8, und
ein Eindrehwerkzeug (100; 100'), aufweisend:
einen ersten Abschnitt (104), der zylindrisch ausgebildet ist, und
einen zweiten Abschnitt (106), der zylindrisch oder kegelstumpfartig ausgebildet ist,
wobei der zweite Abschnitt (106) ferner eine Aufnahme (110) für ein medizinisches Werkzeug, ein chirurgisches Implantat oder einen anderen Gegenstand aufweist.

10. Drehmomentübertragungssystem nach Anspruch 9, **dadurch gekennzeichnet, dass** zwischen der Ratsche (1; 1'; 1 "; 1"') und dem Eindrehwerkzeug (100; 100') eine kraftschlüssige Verbindung vorliegt.

11. Drehmomentübertragungssystem nach Anspruch 9 oder 10, **dadurch gekennzeichnet, dass** zwischen der Ratsche (1; 1'; 1"; 1"') und dem Eindrehwerkzeug (100; 100') eine formschlüssige Verbindung vorliegt.

12. Drehmomentübertragungssystem nach Anspruch 11, **dadurch gekennzeichnet, dass** der erste Abschnitt (104) am Eindrehwerkzeug (100; 100') wenigstens eine entlang von dessen Manteloberfläche (108) angeordnete Ausnehmung (102; 102') aufweist, wobei eine formschlüssige Verbindung dadurch hergestellt wird, dass der wenigstens eine Vorsprung (10; 10.1; 10.2; 10.3; 10.4; 10.5; 10.6; 10.7) der Spirale (6; 6'; 6"; 6"') der Ratsche (1; 1'; 1"; 1"') mit der wenigstens einen Ausnehmung (102; 102') des Eindrehwerkzeug (100; 100') in Eingriff steht.

13. Drehmomentübertragungssystem nach Anspruch 12, **dadurch gekennzeichnet, dass** entlang der Manteloberfläche (108) des Eindrehwerkzeugs (100; 100') eine Mehrzahl der Ausnehmungen (102; 102') mit einem periodischen Abstand angeordnet ist.

14. Verwendung einer Ratsche (1; 1'; 1"; 1"') nach wenigstens einem der Ansprüche 1 bis 8 in der Chirurgie oder im Dentalbereich, zum Übertragen eines Drehmoments auf ein Eindrehwerkzeug (100; 100'). \

15. Verfahren zum Übertragen eines aufzubringenden Drehmoments auf ein Eindrehwerkzeug (100; 100') vermittels einer Ratsche (1; 1'; 1"; 1"') nach wenigstens einem der Ansprüche 1 bis 8, mit den Schritten:
- Aufsetzen der Ratsche (1; 1'; 1 "; 1"') auf das Eindrehwerkzeug (100; 100') in dessen axialer Richtung, sowie vorzugsweise Drehen der Ratsche (1; 1'; 1"; 1"') in eine Freilaufrichtung bei gleichzeitigem Absenken der Ratsche (1; 1'; 1"; 1"') entlang des Eindrehwerkzeugs (100; 100');
- Fixieren der Ratsche (1; 1'; 1"; 1"') auf dem Eindrehwerkzeug (100; 100') und Übertragen des Drehmoments auf das Eindrehwerkzeug (100; 100') durch Drehen der Ratsche (1; 1'; 1"; 1"') in eine Drehmomentübertragungsrichtung;
- Freigeben des Eindrehwerkzeugs (100; 100') durch Drehen der darauf befindlichen Ratsche (1; 1'; 1"; 1'") in Freilaufrichtung;
- Lösen der Ratsche (1; 1'; 1 "; 1"') vom Eindrehwerkzeug (100; 100') durch Drehen in Freilaufrichtung mit gleichzeitigem Abnehmen der Ratsche (1; 1'; 1"; 1"') vom Eindrehwerkzeug (100; 100') in dessen axialer Richtung.

16. Verfahren nach Anspruch 15, **dadurch gekennzeichnet, dass** der Schritt
- Fixieren der Ratsche (1; 1'; 1"; 1"') auf dem Eindrehwerkzeug (100; 100') und Übertragen des Drehmoments auf das Eindrehwerkzeug (100; 100') durch Drehen der Ratsche (1; 1'; 1 "; 1"') in Drehmomentübertragungsrichtung wiederholt ausgeführt wird, und
dass der Schritt
- Freigeben des Eindrehwerkzeugs (100; 100') durch Drehen der darauf befindlichen Ratsche (1; 1'; 1 "; 1"') in Freilaufrichtung wiederholt ausgeführt wird, bevor die Ratsche (1; 1'; 1"; 1"') vom Eindrehwerkzeug (100; 100') gelöst wird.

17. Verfahren nach Anspruch 15 oder 16, **dadurch gekennzeichnet, dass** der Schritt
- Fixieren der Ratsche (1; 1'; 1"; 1"') auf dem Eindrehwerkzeug (100; 100') und Übertragen des Drehmoments auf das Eindrehwerkzeug (100; 100') durch Drehen der Ratsche (1; 1'; 1"; 1"') in Drehmomentübertragungsrichtung so ausgeführt wird, dass wenigstens ein Vorsprung (10; 10.1; 10.2; 10.3; 10.4; 10.5; 10.6; 10.7) einer Spirale (6; 6'; 6"; 6"') mit wenigstens einer Ausnehmung (102; 102') des Eindrehwerkzeugs (100; 100') in Eingriff steht;
und der Schritt
- Freigeben des Eindrehwerkzeugs (100; 100') durch Drehen der darauf befindlichen Ratsche (1; 1'; 1 "; 1"') in Freilaufrichtung so ausgeführt wird,
dass sich der wenigstens eine Vorsprung (10; 10.1; 10.2; 10.3; 10.4; 10.5; 10.6; 10.7) der Spirale (6; 6'; 6"; 6"') von der wenigstens einen Ausnehmung (102; 102') des Eindrehwerkzeugs (100; 100') löst.

18. Verfahren nach Anspruch 15 oder 16, **dadurch gekennzeichnet ist, dass** der Schritt
- Fixieren der Ratsche (1; 1'; 1 "; 1"') auf dem Eindrehwerkzeug (100; 100') und Übertragen des Drehmoments auf das Eindrehwerkzeug (100; 100') durch Drehen der Ratsche (1; 1'; 1"; 1"') in Drehmomentübertragungsrichtung so ausgeführt wird, dass ein erster Teil des an dem Drehpunktgehäuse (4; 4'; 4"; 4'") aufgebrachten Drehmoments die biegeelastische Spirale (6; 6'; 6"; 6"') weiter in einer Ebene eindreht, so dass zwischen einer der Drehachse (D) zugewandten Flanke (8; 8') und dem Eindrehwerkzeug (100; 100') eine kraftschlüssige Verbindung vorliegt, und
dass ein zweiter Teil des an dem Drehpunktgehäuse (4; 4'; 4"; 4"') aufgebrachten Drehmoments eine Drehbewegung des Eindrehwerkzeugs (100; 100') verursacht;
und der Schritt
- Freigeben des Eindrehwerkzeugs (100; 100') durch Drehen der darauf befindlichen Ratsche (1; 1'; 1 "; 1"') in Freilaufrichtung so ausgeführt wird,
dass sich die kraftschlüssige Verbindung zwischen der der Drehachse (D) zugewandten Flanke (8; 8') der Spirale (6; 6'; 6"; 6"') und dem Eindrehwerkzeug (100; 100') löst.

19. Verfahren zum Herstellen einer Ratsche (1; 1'; 1"; 1"') nach wenigstens einem der Ansprüche 1 bis 8 mit den Schritten:
Auswählen eines Rohlings;
Ausbilden einer Kontur der Ratsche (1; 1'; 1 "; 1"') vermittels Laserschneiden, Wasserstrahlschneiden, Drahterodieren, Funkenerodieren oder Stanzen.

20. Verfahren zum Herstellen einer Ratsche (1; 1'; 1"; 1"') nach Anspruch 19, **dadurch gekennzeichnet, dass** der Rohling eine einheitliche Dicke (d) aufweist.

## Claims

1. A ratchet (1; 1'; 1"; 1"') for the medical field, comprising:
a handle (2; 2'; 2"; 2"'), and
a pivot housing (4; 4'; 4"; 4"') arranged thereon,
**characterized in that**
the pivot housing (4; 4'; 4"; 4"') is a flat spiral (6; 6'; 6"; 6"'),
wherein the spiral (6; 6'; 6"; 6"') is flexible,
wherein the ratchet (1; 1'; 1 "; 1"') can be actuated such that the spiral (6; 6'; 6"; 6"') coils up in a plane that is oriented substantially vertically to the pivot axis (D), and wherein
the spiral (6, 6', 6", 6"') is designed as a logarithmic spiral, a hyperbolic spiral or an archimedic spiral.

2. The ratchet according to claim 1, **characterized in that** it is of single-piece design, preferably formed of stainless steel, titanium, ceramics, wear-proof plastics, or the like.

3. The ratchet according to claims 1 or 2, **characterized in that** the spiral (6; 6'; 6"; 6"') has an angle of at least 360 °, preferably of at least 450 °, particularly preferred of at least 630 °.

4. The ratchet according to any of claims 1 to 3, **characterized in that** the spiral (6; 6'; 6"; 6"') has at least one projection (10; 10.1; 10.2; 10.3; 10.4; 10.5; 10.6; 10.7) facing the pivot axis (D), wherein the projection (10; 10.1; 10.2; 10.3; 10.6; 10.7) facing the pivot axis (D) is preferably arranged on a free end (12) of the spiral (6; 6'; 6"; 6"'), and wherein a surface (11.6) of the projection (10.6) which faces away from the free end (12) of the spiral (6"") preferably forms an angle (α) with an edge (8; 8') facing the pivot axis (D).

5. The ratchet according to claim 4, **characterized in that** the spiral (6; 6'; 6"; 6"') has a recess (13.2) extending across a length (L),
wherein the recess (13.2) directly adjoins the surface (11.6) of the projection (10.6) which faces away from the free end (12) of the spiral (6"").

6. The ratchet according to any of claims 1 to 5, **characterized in that** the edge (8; 8') of the spiral (6; 6'; 6"; 6"') facing the pivot axis (D) is roughened in at least one section and/or that the edge (8; 8') of the spiral (6; 6'; 6"; 6"') facing the pivot axis (D) is hardened in at least one section and/or that the at least one projection (10; 10.1; 10.2; 10.3; 10.4; 10.5; 10.6; 10.7) is hardened in at least one section.

7. The ratchet according to any of claims 1 to 6, **characterized in that** a marking (3) is provided in the region of the handle (2; 2'; 2"; 2"') and/or the pivot housing (4; 4'; 4"; 4"'), wherein the handle (2; 2'; 2"; 2"') and the pivot housing (4; 4'; 4"; 4"') preferably have the same thickness (d) and/or the handle (2; 2'; 2"; 2"') is preferably cylindrical.

8. The ratchet according to any of claims 1 to 7, **characterized in that** it comprises a stop (14) projecting at least partially into the region of the spiral center.

9. A torque transmission system, comprising:
a ratchet (1; 1'; 1"; 1"') according to at least any of claims 1 to 8, and an implant driver (100; 100'), comprising:
a first section (104) of cylindrical design, and
a second section (106) of cylindrical or frustoconical design, wherein the second section (106) further comprises a receptacle (110) for a medical tool, a surgical implant, or any other object.

10. The torque transmission system according to claim 9, **characterized in that** a force-fit connection exists between the ratchet (1; 1'; 1"; 1"') and the implant driver (100; 100').

11. The torque transmission system according to claim 9 or 10, **characterized in that** a form-fit connection exists between the ratchet (1; 1'; 1"; 1"') and the implant driver (100; 100').

12. The torque transmission system according to claim 11, **characterized in that** the first section (104) on the implant driver (100; 100') comprises at least one recess (102; 102') arranged along the shell surface (108) thereof, wherein a form-fit connection is established by the at least one projection (10; 10.1; 10.2; 10.3; 10.4; 10.5; 10.6; 10.7) of the spiral (6; 6'; 6"; 6"') of the ratchet (1; 1'; 1"; 1"') being in engagement with the at least one recess (102; 102') of the implant driver (100; 100').

13. The torque transmission system according to claim 12, **characterized in that** a plurality of the recesses (102; 102') are arranged with periodic distances along the shell surface (108) of the implant driver (100; 100').

14. A use of a ratchet (1; 1'; 1"; 1"') according to at least any of claims 1 to 8 in surgery or in the dental field, for transmitting a torque to an implant driver (100; 100').

15. A method for transmitting a torque to be applied to an implant driver (100; 100') by means of a ratchet (1; 1'; 1"; 1"') according to at least any of claims 1 to 8, comprising the steps of:
- placing the ratchet (1; 1'; 1"; 1"') on the implant driver (100; 100') in the axial direction thereof, and preferably rotating the ratchet (1; 1'; 1"; 1"') in a freewheeling direction while simultaneously lowering the ratchet (1; 1'; 1"; 1"') along the implant driver (100; 100');
- fixing the ratchet (1; 1'; 1"; 1"') on the implant driver (100; 100') and transmitting the torque to the implant driver (100; 100') by rotating the ratchet (1; 1'; 1"; 1"') in the direction of torque transmission;
- releasing the implant driver (100; 100') by rotating the ratchet (1; 1'; 1"; 1'") positioned thereon in freewheeling direction;
- detaching the ratchet (1; 1'; 1"; 1"') from the implant driver (100; 100') by rotating in freewheeling direction while simultaneously removing the ratchet (1; 1'; 1"; 1"') from the implant driver (100; 100') in the axial direction thereof.

16. The method according to claim 15, **characterized in that** the step
- fixing the ratchet (1; 1'; 1"; 1"') on the implant driver (100; 100') and transmitting the torque to the implant driver (100; 100') by rotating the ratchet (1; 1'; 1"; 1"') in the direction of torque transmission is performed repeatedly, and
that the step
- releasing the implant driver (100; 100') by rotating the ratchet (1; 1'; 1"; 1"') positioned thereon in freewheeling direction is performed repeatedly, before the ratchet (1; 1'; 1"; 1"') is detached from the implant driver (100; 100').

17. The method according to claim 15 or 16, **characterized in that** the step
- fixing the ratchet (1; 1'; 1"; 1"') on the implant driver (100; 100') and transmitting the torque to the implant driver (100; 100') by rotating the ratchet (1; 1'; 1"; 1"') in the direction of torque transmission is performed such that at least one projection (10; 10.1; 10.2; 10.3; 10.4; 10.5; 10.6; 10.7) of a spiral (6; 6'; 6"; 6"') is in engagement with at least one recess (102; 102') of the implant driver (100; 100');
and that the step
- releasing the implant driver (100; 100') by rotating the ratchet (1; 1'; 1"; 1"') positioned thereon in freewheeling direction is performed such that the at least one projection (10; 10.1; 10.2; 10.3; 10.4; 10.5; 10.6; 10.7) of the spiral (6; 6'; 6"; 6"') disengages from the at least one recess (102; 102') of the implant driver (100; 100').

18. The method according to claim 15 or 16, **characterized in that** the step
- fixing the ratchet (1; 1'; 1"; 1"') on the implant driver (100; 100') and transmitting the torque to the implant driver (100; 100') by rotating the ratchet (1; 1'; 1"; 1"') in the direction of torque transmission is performed such that a first part of the torque applied to the pivot housing (4; 4'; 4"; 4"') continues to coil up the flexible spiral (6; 6'; 6"; 6"') in a plane, so that a force-fit connection exists between an edge (8; 8') facing the pivot axis (D) and the implant driver (100; 100'), and that a second part of the torque applied to the pivot housing (4; 4'; 4"; 4"') causes a pivoting movement of the implant driver (100; 100');
and that the step
- releasing the implant driver (100; 100') by rotating the ratchet (1; 1'; 1"; 1"') positioned thereon in freewheeling direction is performed such that the force-fit connection between the edge (8; 8') of the spiral (6; 6'; 6"; 6"') facing the pivot axis (D) and the implant driver (100; 100') is released.

19. A method for manufacturing a ratchet (1; 1'; 1"; 1"') according to at least any of claims 1 to 8, comprising the steps of:
selecting a blank;
forming a contour of the ratchet (1; 1'; 1"; 1"') by means of laser cutting, water jet cutting, wire eroding, spark eroding, or punching.

20. The method for manufacturing a ratchet (1; 1'; 1"; 1"') according to claim 19, **characterized in that** the blank has a uniform thickness (d).

## Revendications

1. Dispositif à cliquet (1 ; 1' ; 1" ; 1"') destiné au domaine médical, présentant :
une poignée (2 ; 2' ; 2" ; 2"'), et
un logement de pivot (4 ; 4' ; 4" ; 4"') disposé au niveau de ladite poignée,
**caractérisé en ce que**
le logement de pivot (4 ; 4' ; 4" ; 4"') est réalisé sous la forme d'une spirale (6 ; 6' ; 6" ; 6"') plane,
sachant que la spirale (6 ; 6' ; 6" ; 6"') est réalisée de manière élastique en flexion,
sachant que le dispositif à cliquet (1 ; 1' ; 1" ; 1"') peut être actionné de telle manière que la spirale (6 ; 6' ; 6" ; 6"') est vissée dans un plan orienté essentiellement de manière perpendiculaire par rapport à l'axe de rotation (D), et sachant que
la spirale (6 ; 6' ; 6" ; 6"') est réalisée sous la forme d'une spirale logarithmique, d'une spirale hyperbolique ou d'une spirale d'Archimède.

2. Dispositif à cliquet selon la revendication 1, **caractérisé en ce qu'**il est réalisé d'un seul tenant de préférence en acier inoxydable, en titane, en céramique, en matières plastiques résistantes à l'usure ou similaires.

3. Dispositif à cliquet selon la revendication 1 ou 2, **caractérisé en ce que** la spirale (6 ; 6' ; 6" ; 6"') présente un angle d'au moins 360°, de préférence d'au moins 450°, de manière particulièrement préférée d'au moins 630°.

4. Dispositif à cliquet selon l'une quelconque des revendications 1 à 3, **caractérisé en ce que** la spirale (6 ; 6' ; 6" ; 6"') présente au moins une partie faisant saillie (10 ; 10.1 ; 10.2 ; 10.3 ; 10.4 ; 10.5 ; 10.6 ; 10.7) tournée vers l'axe de rotation (D), sachant que la partie faisant saillie (10 ; 10.1 ; 10.2 ; 10.3 ; 10.4 ; 10.5 ; 10.6 ; 10.7) tournée vers l'axe de rotation (D) est disposée de préférence au niveau d'une extrémité libre (12) de la spirale (6 ; 6' ; 6" ; 6"') et sachant qu'une surface (11.6), opposée à l'extrémité libre (12) de la spirale (6""), de la partie faisant saillie (10.6) forme avec un flanc (8 ; 8') tourné vers l'axe de rotation (D), de préférence un angle (α).

5. Dispositif à cliquet selon la revendication 4, **caractérisé en ce que** la spirale (6 ; 6' ; 6" ; 6"') présente un évidement (13.2) s'étendant sur une longueur (L), sachant que l'évidement (13.2) est adjacent à la surface (11.6), opposée à l'extrémité libre (12) de la spirale (6""), de la partie faisant saillie (10.6).

6. Dispositif à cliquet selon l'une quelconque des revendications 1 à 5, **caractérisé en ce que** le flanc (8 ; 8') tourné vers l'axe de rotation (D), de la spirale (6 ; 6' ; 6" ; 6"') est rendu rugueux au moins dans une section, et/ou **en ce que** le flanc (8 ; 8'), tourné vers l'axe de rotation (D), de la spirale (6 ; 6' ; 6" ; 6"') est réalisé de manière durcie au moins dans une section, et/ou la partie faisant saillie (10 ; 10.1 ; 10.2 ; 10.3 ; 10.4 ; 10.5 ; 10.6 ; 10.7) au moins au nombre de une est réalisée de manière durcie au moins dans une section.

7. Dispositif à cliquet selon l'une quelconque des revendications 1 à 6, **caractérisé en ce qu'**un marquage (3) est prévu dans la zone de la poignée (2 ; 2' ; 2" ; 2"') et/ou du logement de pivot (4 ; 4' ; 4" ; 4"'), sachant que la poignée (2 ; 2' ; 2" ; 2"') et le logement de pivot (4 ; 4' ; 4" ; 4"') présentent de préférence la même épaisseur (d) et/ou la poignée (2 ; 2' ; 2" ; 2"') est réalisée de préférence de manière cylindrique.

8. Dispositif selon l'une quelconque des revendications 1 à 7, **caractérisé en ce qu'**il présente une butée (14), qui dépasse au moins en partie dans la zone du centre de spirale.

9. Système de transmission de couple de rotation, présentent :
un dispositif à cliquet (1 ; 1' ; 1" ; 1"') selon au moins l'une quelconque des revendications 1 à 8, et
un outil de vissage (100 ; 100'), présentant :
une première section (104) qui est réalisée de manière cylindrique, et
une deuxième section (106) qui est réalisée de manière cylindrique ou à la manière d'un cône tronqué,
sachant que la deuxième section (106) présente en outre un logement (110) destiné à un outil médical, un implant chirurgical ou un autre objet.

10. Système de transmission de couple de rotation selon la revendication 9, **caractérisé en ce qu'**une liaison à force est présente entre le dispositif à cliquet (1 ; 1' ; 1" ; 1"') et l'outil de vissage (100 ; 100').

11. Système de transmission de couple de rotation selon la revendication 9 ou 10, **caractérisé en ce qu'**une liaison par complémentarité de forme est présente entre le dispositif à cliquet (1 ; 1' ; 1" ; 1"') et l'outil de vissage (100 ; 100').

12. Système de transmission de couple de rotation selon la revendication 11, **caractérisé en ce que** la première section (104) au niveau de l'outil de vissage (1000 ; 100') présente au moins un évidement (102 ; 102') disposé le long de la surface extérieure (108) de ladite section, sachant qu'une liaison par complémentarité de forme est établie **en ce que** la partie faisant saillie (10 ; 10.1 ; 10.2 ; 10.3 ; 10.4 ; 10.5 ; 10.6 ; 10.7) de la spirale (6 ; 6' ; 6" ; 6"') du dispositif à cliquet (1 ; 1' ; 1" ; 1"') est en prise avec l'évidement (102 ; 102') au moins au nombre de un de l'outil de vissage (100 ; 100').

13. Système de transmission de couple de rotation selon la revendication 12, **caractérisé en ce qu'**une majorité des évidements (102 ; 102') est disposée à une distance périodique le long de la surface extérieure (108) de l'outil de vissage (100 ; 100').

14. Utilisation d'un dispositif à cliquet (1 ; 1' ; 1" ; 1"') selon au moins l'une quelconque des revendications 1 à 8 en chirurgie ou dans le domaine dentaire, servant à transmettre un couple de rotation sur un outil de vissage (100 ; 100').

15. Procédé servant à transmettre un couple de rotation à appliquer sur un outil de vissage (100 ; 100') par l'entremise d'un dispositif à cliquet (1 ; 1' ; 1" ; 1"') selon au moins l'une quelconque des revendications 1 à 8, comprenant les étapes suivantes consistant à :
- poser le dispositif à cliquet (1 ; 1' ; 1" ; 1"') sur l'outil de vissage (100 ; 100') dans sa direction axiale, ainsi que de préférence faire tourner le dispositif à cliquet (1 ; 1' ; 1" ; 1"') dans une direction de roue libre tout en abaissant dans le même temps le dispositif à cliquet (1 ; 1' ; 1" ; 1"') le long de l'outil de vissage (100 ; 100') ;
- fixer le dispositif à cliquet (1 ; 1' ; 1" ; 1"') sur l'outil de vissage (100 ; 100') et transmettre le couple de rotation sur l'outil de vissage (100 ; 100') par la rotation du dispositif à cliquet (1 ; 1' ; 1" ; 1"') dans une direction de transmission de couple de rotation ;
- desserrer l'outil de vissage (100 ; 100') par la rotation du dispositif à cliquet (1 ; 1' ; 1" ; 1"') se trouvant sur ce dernier dans la direction de roue libre ;
- détacher le dispositif à cliquet (1 ; 1' ; 1" ; 1"') de l'outil de vissage (100 ; 100') par la rotation dans la direction de roue libre tout en retirant dans le même temps le dispositif à cliquet (1 ; 1' ; 1" ; 1"') de l'outil de vissage (100 ; 100') dans sa direction axiale.

16. Procédé selon la revendication 15, **caractérisé en ce que** l'étape
- consistant à fixer le dispositif à cliquet (1 ; 1' ; 1" ; 1"') sur l'outil de vissage (100 ; 100') et à transmettre le couple de rotation sur l'outil de vissage (100 ; 100') par la rotation du dispositif à cliquet (1 ; 1' ; 1" ; 1"') sont exécutées de manière répétée dans la direction de transmission de couple de rotation, et
**en ce que** l'étape
- consistant à desserrer l'outil de vissage (100 ; 100') par la rotation du dispositif à cliquet (1 ; 1' ; 1" ; 1"') se trouvant sur ce dernier est exécutée de manière répétée dans la direction de roue libre, avant que le dispositif à cliquet (1 ; 1' ; 1" ; 1"') ne soit détaché de l'outil de vissage (100 ; 100').

17. Procédé selon la revendication 15 ou 16, **caractérisé en ce que** l'étape
- consistant à fixer le dispositif à cliquet (1 ; 1' ; 1" ; 1"') sur l'outil de vissage (100 ; 100') et à transmettre le couple de rotation sur l'outil de vissage (100 ; 100') par la rotation du dispositif à cliquet (1 ; 1' ; 1" ; 1"') sont exécutées dans la direction de transmission de couple de rotation de telle manière
qu'au moins une partie faisant saillie (10 ; 10.1 ; 10.2 ; 10.3 ; 10.4 ; 10.5 ; 10.6 ; 10.7) d'une spirale (6 ; 6' ; 6" ; 6"') est en prise avec au moins un évidement (102 ; 102') de l'outil de vissage (100 ; 100' ;
et **en ce que** l'étape
- consistant à desserrer l'outil de vissage (100 ; 100') par la rotation du dispositif à cliquet (1 ; 1' ; 1" ; 1"') se trouvant sur ce dernier est exécutée dans la direction roue mobile de telle manière
que la partie faisant saillie (10 ; 10.1 ; 10.2 ; 10.3 ; 10.4 ; 10.5 ; 10.6 ; 10.7) au moins au nombre de une de la spirale (6 ; 6' ; 6" ; 6"') se desserre de l'évidement (102 ; 102') au moins au nombre de un de l'outil de vissage (100 ; 100').

18. Procédé selon la revendication 15 ou 16, **caractérisé en ce que** l'étape
- consistant à fixer le dispositif à cliquet (1 ; 1' ; 1" ; 1"') sur l'outil de vissage (100 ; 100') et à transmettre le couple de rotation sur l'outil de vissage (100 ; 100') par la rotation du dispositif à cliquet (1 ; 1' ; 1" ; 1"') sont exécutées de telle sorte
qu'une première partie du couple de rotation appliquée sur le logement de pivot (4 ; 4' ; 4" ; 4"') continue à visser, dans un plan, la spirale (6 ; 6' ; 6" ; 6"') élastique en flexion de sorte qu'une liaison à force est présente entre un flanc (8 ; 8') tourné vers l'axe de rotation (D) et l'outil de vissage (100 ; 100'), et
qu'une deuxième partie du couple de rotation appliqué au niveau du logement de pivot (4 ; 4' ; 4" ; 4"') entraîne un mouvement de rotation de l'outil de vissage (100 ; 100') ;
et **en ce que** l'étape
- consistant à desserrer l'outil de vissage (100 ; 100') par la rotation du dispositif à cliquet (1 ; 1' ; 1" ; 1"') se trouvant sur ce dernier dans la direction de roue libre de telle manière
que la liaison à force se déclenche entre le flanc (8 ; 8') tourné vers l'axe de rotation (D) de la spirale (6 ; 6' : 6" ; 6"') et l'outil de vissage (100 ; 100').

19. Procédé servant à fabriquer un dispositif à cliquet (1 ; 1' ; 1" ; 1"') selon au moins l'une quelconque des revendications 1 à 8, comprenant les étapes consistant à
sélectionner une ébauche ;
former un contour du dispositif à cliquet (1 ; 1' ; 1" ; 1"') par l'entremise d'un découpage au laser, d'un découpage par jet d'eau, par étincelage par fil, par électroérosion ou par estampage.

20. Procédé servant à fabriquer un dispositif à cliquet (1 ; 1' ; 1" ; 1"') selon la revendication 19, **caractérisé en ce que** l'ébauche présente une épaisseur uniforme (d).
